# EUROPEAN PATENT APPLICATION

(11) **EP 3 928 728 A1**
(43) Date of publication of application: **29.12.2021**
(21) Application number: 21180616.1
(22) Date of filing: 21.06.2021
(51) Int. Cl.: A61B 17/74

(54) **ASSEMBLED INTRAMEDULLARY NAIL SYSTEM**

(30) Priority: 22.06.2020 CN 202010571723
(71) Applicant: Changzhou Kanghui Medical Innovation Co., Ltd., Changzhou, Jiangsu (CN)
(72) Inventor: QIN, Zongjun, Changzhou, 213100 (CN); LI, Yuanxun, Shanghai, 200333 (CN); PEI, Lei, Changzhou, 213100 (CN); YANG, Zhaoquan, Changzhou, 213100 (CN); LIU, Bo, Changzhou, 213100 (CN); KE, Linhua, Changzhou, 213100 (CN); GUAN, Jianjun, Changzhou, 213100 (CN)
(74) Representative: Müller-Boré & Partner Patentanwälte PartG mbB

(57) **Abstract**

The present disclosure relates to an assembled intramedullary nail system having a simple structure and enabling various assembling manners. The assembled intramedullary nail system includes a main nail for intramedullary nail, a set screw, a lag screw, a compression screw, a locking screw, and a cap. The main nail for intramedullary nail provides a respective installment space for various components by means of a center through-hole, an overlapping hole disposed at a proximal end, and a locking screw hole disposed at a distal end. The main nail for intramedullary nail, the lag screw, the compression screw, the locking screw and the cap are respectively of multiple specifications which are different preferably in diameter or length. The set screw is pre-installed within the main screw. Based on the differences of the spiral structures at the head portion, the lag screw can be classified into two types, namely a spiral blade type and a threaded type. The compression screw is optional, which is pre-installed within the lower hole of the overlapping hole at the proximal end of the main nail. By rotating the compression screw, the lag screw slides rearwards along its axial direction. Moreover, the combination of the compression screw and the lag screw optimizes the anti-rotation performance of the lag screw relative to the femur.

## Description

### FIELD

The present disclosure relates to an assembled intramedullary nail system having a simple structure and enabling various assembling manners.

### BACKGROUND

Intramedullary nailing is the main method for treatment of long bone fractures. In particular, more than 60% of the total amounts of intramedullary nails are applied to treatment of intertrochanteric fractures of thighbone. When choosing intramedullary nailing solutions for intertrochanteric fractures, surgeons typically take fracture types and bone conditions of patients into account. Among the mainstream solutions available on the market, the threaded lag screws are suitable for young patients with good bone conditions while the spiral-blade-type lag screw, owing to poor anti-rotation property at its head, are more suitable for elderly patients with poor bone conditions. It is difficult for the spiral-blade-type lag screw to be knocked into and pulled out from the bone of the young patients. In short, the clinical needs of patients may be personalized according to the different fracture types and bone conditions. Current solutions available in the market have certain limitations. Although it is impossible to provide personalized treatment for each patient, it would still be an improvement to offer the surgeons with an intramedullary nail system having a simple structure but enabling various assembling manners.

### SUMMARY

In connection with the above defects, an objective of the present disclosure is to provide an assembled intramedullary nail system having a simple structure and enabling various assembling manners to satisfy clinical needs of patients suffering from different types of femoral fractures.

The assembled intramedullary nail system comprises: a main nail, a threaded lag screw, a spiral-blade-type lag screw, a long compression screw, a short compression screw, and at least one locking screw. The main nail comprises a center through-hole, an overlapping hole, and at least one locking screw hole corresponding to the locking screw. A side of the overlapping hole adjacent to a proximal end is greater than the other side adjacent to a distal end. The threaded lag screw and the spiral-blade-type lag screw can be interchangeably installed at one side of the overlapping hole adjacent to the proximal end. The long compression screw and the short compression screw can be interchangeably installed at one side of the overlapping hole adjacent to the distal end. The locking screw can be disposed within the locking screw hole.

According to an aspect of the present disclosure, the main nail is a long or short main nail.

According to another aspect of the present disclosure, the assembled intramedullary nail system comprises three locking screws, and the main nail comprises three locking screw holes.

According to a further aspect of the present disclosure, the threaded lag screw and the spiral-blade-type lag screw comprise bone-cement holes.

According to a still further aspect of the present disclosure, the assembled intramedullary nail system further comprises a set screw pre-installed within the center through-hole of the main nail and being adapted to cooperate with an engaging rail of the lag screw.

According to a still further aspect of the present disclosure, by driving the set screw, the threaded lag screw and the spiral-blade-type lag screw are switched between a locked state and an unlocked state.

According to a still further aspect of the present disclosure, the threaded lag screw and the spiral-blade-type screw each comprises a lag thread at a head portion and a compression rail at a middle portion. The long compression screw and the short compression screw each comprises a compression thread at a head portion, and a tail portion of the long compression screw and the short compression screw being a cylinder. The compression threads cooperate with the compression rail. The compression screw is configured to be installed in the overlapping hole and rotated to drive the lag screw to slide in an anterior-posterior direction along its axial direction.

According to a still further aspect of the present disclosure, the lag screw comprises a driving slot at the tail portion.

According to a still further aspect of the present disclosure, the compression rail and the engaging rail are respectively disposed at two sides of the threaded lag screw or the spiral-blade-type lag screw. According to a still further aspect of the present disclosure, the assembled intramedullary nail system further comprises a tail cap which can be installed within the center through-hole at the proximal end of the main nail.

The assembled intramedullary nail system according to the present disclosure can provide various assembling options (in the assembling manners described below, the set screw is pre-installed within the center through-hole of the intramedullary nail system):
According to the differences of the femur bone conditions and fracture types among the patients, the threaded lag screws for the intramedullary nail can be used in combination as required as follows: a. for a stable fracture, the simplest combination including an intramedullary nail, a threaded lag screw, a locking screw, and a cap may be selected, wherein the set screw is in an unlocked state (i.e., it is elastically locked) and thus can only move rearwards, not forwards; b. if the restoration for a fracture is not good, compression is required at the first stage of the surgery, where a compression screw is additionally provided within the overlapping hole below the proximal end of the main nail to achieve a stable controllable compression operation during surgery; c. for a patient having a narrow femoral neck, a short screw in terms of length specification may be selected, such as a short compression screw not passing through the femoral isthmus; and d. for a patient having a poor bone condition, a lag screw having bone-cement through-holes at the head portion may be selected, wherein bone cement is injected to achieve stabilization of the prosthesis and the patient's femur at the first stage.

According to the differences of the femur bone conditions and fracture types among the patients, the spiral-blade-type lag screws for the intramedullary nail can be used in combination as required as follows: a. for a stable fracture, the simplest combination including an intramedullary nail, a spiral-blade-type lag screw, a locking screw, and a cap may be selected, wherein the set screw is in an unlocked state (i.e., it is elastically locked) and thus can only move rearwards, not forwards; b. if the restoration for a fracture is not good, compression is required at the first stage the surgery, wherein a compression screw is additionally provided within the overlapping hole below the proximal end of the main nail to achieve a stable controllable compression operation during surgery; c. for a patient having a narrow femoral neck, a short screw in terms of length specification may be selected, such as a short compression screw not passing through the femoral isthmus; and d. for a patient having a poor bone condition, a spiral-blade-type lag screw having bone-cement through-holes at the head portion may be selected, wherein bone cement is injected to achieve stabilization of the prosthesis and the patient's femur at the first stage.

The advantages of the assembled intramedullary nail system have been described above. In clinic, such an assembled femoral intramedullary nail system can provide multiple flexible femoral intramedullary nailing solutions, to allow surgeons to implement free assembling according to specific clinical needs. The solution according to the present disclosure can satisfy fixing needs for nine types of AO proximal femoral fractures.

The present disclosure provides an intramedullary femoral nail system having a simple structure and enabling various flexible assembling manners to meet clinical needs for treating patients suffering from different femoral fractures.

### BRIEF DESCRIPTION OF THE DRAWINGS

The present disclosure will be described below with reference to the drawings, where:
Fig. 1 is a schematic diagram illustrating an assembled intramedullary nail system;
Fig. 2 is a schematic diagram illustrating a structure of an assembled intramedullary nail system;
Fig. 3 is a sectional view of a main nail for an assembled intramedullary nail system;
Fig. 4 is a schematic diagram of respective structures of a spiral-blade-type lag screw and a threaded lag screw for an assembled intramedullary nail system;
Fig. 5 is schematic diagram illustrating structures of lag screws with bone-cement through-hole;
Fig. 6 is a schematic diagram illustrating a structure of a compression screw for an assembled intramedullary nail system;
Fig. 7 is a schematic diagram illustrating different assembling manners when a main nail in an assembled intramedullary nail system is a short nail, where a respective part is of multiple specifications;
Fig. 8 is a diagram illustrating AO fracture types.

The drawings contain the following reference symbols for respective features, including:
- 1: main nail,
- 2: set screw,
- 3: lag screw,
- 4: compression screw,
- 5: locking screw,
- 6: cap,
- 1-1: center through-hole,
- 1-2: overlapping hole,
- 1-3: locking screw hole,
- 1-4: step,
- 1-5: chute,
- 31: lag threads,
- 32: compression rail,
- 33: driving slot,
- 34: engaging rail,
- 35: bone-cement through-hole,
- 41: head threads,
- 42: cylinder.

### DETAILED DESCRIPTION OF EMBODIMENTS

Hereinafter, reference will be made to the drawings to specifically describe the technical solution according to the present disclosure, and the embodiments as described herein are only part of the embodiments of the present disclosure, rather than all of them. Other embodiments that those skilled in the art would acquire without doing creative work, on the basis of the embodiments as described herein, should all fall into the protection scope of the present disclosure.

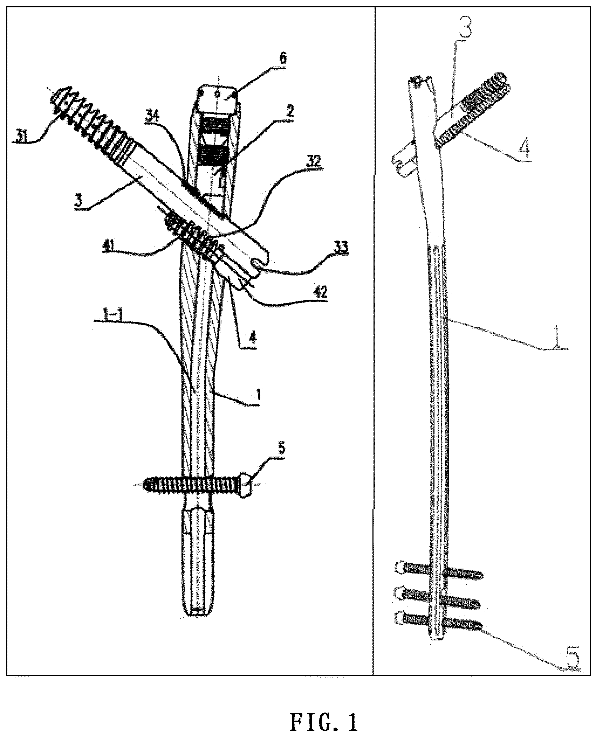

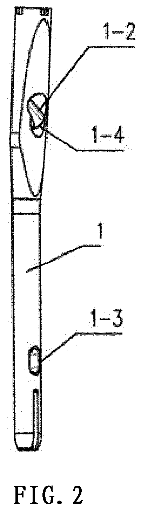

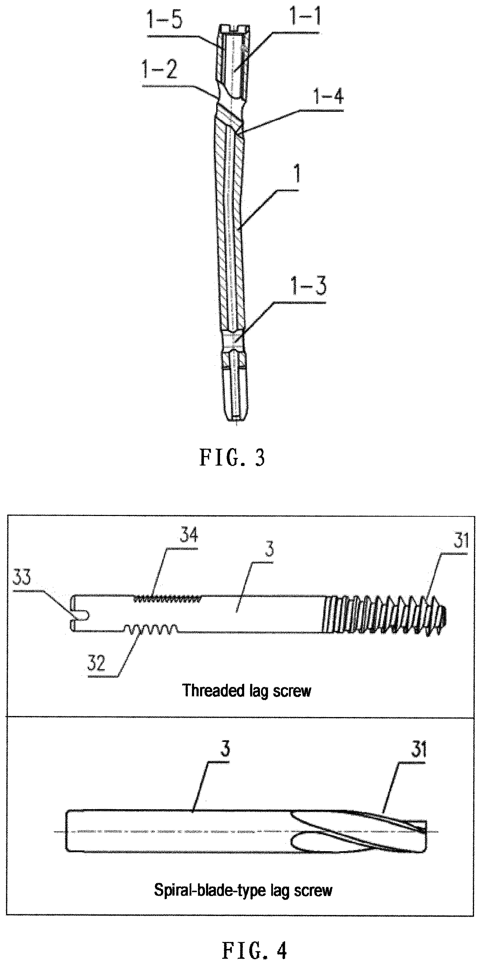

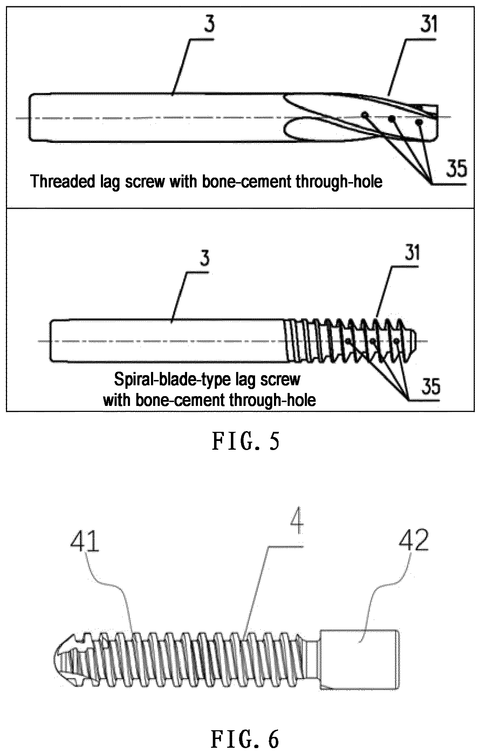

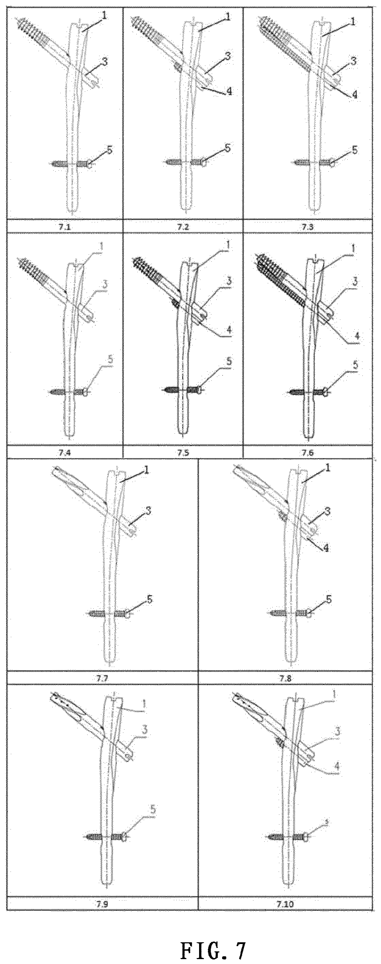

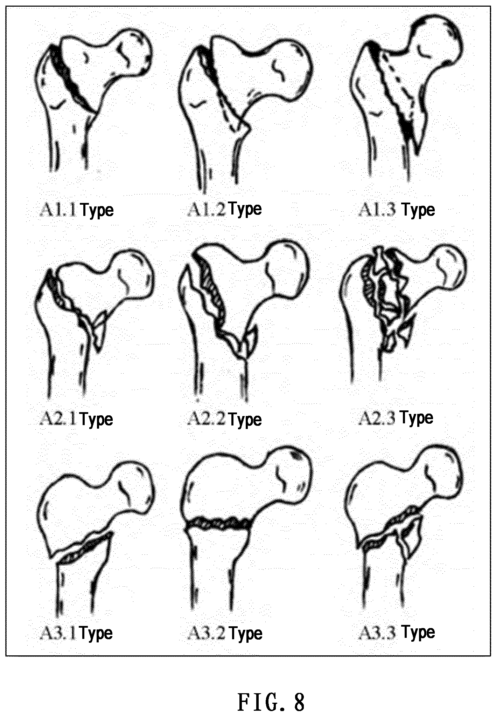

Referring to Figs. 1-8, the main nail for an intramedullary nail system includes six main parts, i.e., a main nail 1, a set screw 2 (pre-installed within the main nail), a lag screw 3, a compression screw 4, a locking screw 5, and a cap 6. The main nail 1 provides a respective installment space for various components by means of a center through-hole 1, an overlapping hole 1-2 disposed at a proximal end, and a locking screw hole 1-3 disposed at a distal end. The main nail 1, the lag screw 3, the compression screw 4, the locking screw 5, and the cap 6 are of multiple specifications which are different preferably in diameter or length. The set screw 2 is pre-installed within the main screw.

The main screw for an intramedullary nail system is classified into a long screw and a short screw. The short screw has a length of 150 mm to 240 mm, preferably not distinguished in terms of left and right, and selectively distinguished in terms of left and right for the specification having a length greater than 200 mm. The long screw has a length of 300 mm to 500 mm. Due to difference in anterior arc, the long screws identical in length have two specifications, namely a left type and a right type. The radius of the anterior arc with a different length is between 1.2 m and 1.6 m.

The center of the main nail is a through-hole. The outer contour of the proximal end of the intramedullary nail is thicker and of a straight shape, and the counterpart of the distal end of the intramedullary nail is thinner and of an arc or a straight shape. An axis of the proximal end contains an angle of 0 to 8 degrees relative to an axis of the distal end. The main nail 1 is transitioned smoothly from the proximal end to the distal end. The intramedullary nail at the proximal and distal sidewalls is provided with at least one hole for accommodating a lag screw, a compression screw, or a fixing and locking screw, respectively.

The main nail is provided with an oblique overlapping hole 1-2 at the proximal end. The overlapping hole 1-2 is characterized in that: a side of the overlapping hole 1-2 adjacent to the proximal end is greater than the other side adjacent to the distal end (i.e., so-called "tapered from top to bottom"). A threaded lag screw and a spiral-blade-type lag screw can be interchangeably installed at the side of the overlapping hole 1-2 adjacent to the proximal end (i.e., the so-called "upper hole" in which a lag screw 3 can be installed), and a long compression screw and a short compression screw can be interchangeably installed at the side of the overlapping hole 1-2 adjacent to the distal end (i.e., the so-called "lower hole" in which the compression screw 4 can be installed, the "lower hole" of the overlapping hole 1-2 is a stepped hole for cooperating with a cylinder 42 of the compression screw 4). The axis of the overlapping hole 1-2 contains an angle of 120 to 140 degrees relative to the axis of the proximal end of the main nail 1.

The main nail is designed with at least one locking screw hole 1-3 at the distal end. A short screw is preferably designed with a locking screw hole 1-3 in a shape of an elongated slot, and a long screw is preferably designed with three locking screw holes 1-3 at the distal end, wherein two locking screw holes 1-3 at the distal end are identical in orientation, and the axis of the remaining locking screw hole 1-3 contains an angle of 10 to 25 degrees relative to the axes of two locking screw holes 1-3. In particular, the furthest locking screw hole 1-3 of the long screw is located in a cancellous bone area and preferably provided with threads.

Based on the differences of lag threads 31 at the head portion, the lag screw 3 can be classified into two types, namely a spiral blade type and a threaded type. Based on presence or absence of bone-cement through-holes 35 at the sidewall of the head spiral area, the lag screw 3 is classified into two types, namely a standard type and a bone cement type. The lag screw 3 at the tail portion is designed with a driving slot 33 for providing a rotational power. The lag screw 3 is designed with a compression rail 32 and an engaging rail 34 at the sidewall of the rear half section. The compression rail 32 and the engaging rail 34 are disposed at two sides of the lag screw, where the compression rail 32 is preferably of a threaded type, and the engaging rail 34 is preferably of a toothed row structure with a negative angle.

The set screw 2 is pre-installed within the center through-hole 1-1 of the main nail 1. The set screw 2 in the middle is provided with an elastic structure. The set screw 2 is driven by an instrument to slide up and down along the chute 1-5. By setting the position of the set screw, the lag screw 3 can be placed into a locked state or an unlocked state.

The compression screw 4 is provided with threads 41 at the head portion and a cylinder 42 at the tail portion, wherein the threads 41 at the head cooperate with the compression rail 32 in the tail area of the lag screw. The lag screw 3 is driven to slide when the compression screw 4 is rotated. The diameter of the tail cylinder 42 is greater than the diameter of the head threads 41. The cylinder 42 cooperates with a step 1-4 in the lower hole of the overlapping hole 1-2, to achieve the effect of rotating in place at a certain fixed position on the main nail 1 and further drive the lag screw 3 to slide in an anterior-posterior direction. Moreover, a connection feature, preferably in the form of hexagonal counterbore, for cooperating with a surgical instrument is provided at the tail end of the cylinder 42. In addition, the combination of the compression screw 4 and the lag screw 3 optimizes the anti-rotation performance of the lag screw 3 in the femur. The cross section formed after the compression screw 4 and the lag screw 3 being in screw fit has two intersecting arcs. The combination having a cross section of two intersecting arcs exhibits a better anti-rotation performance in the femur than a single lag screw having a round cross section.

The locking screw 5 is used to be installed within the locking screw hole 1-3 at the distal end of the main nail 1. The locking screw 5 has two fixed modes in the elongated locking screw hole 1-3 at the distal end of the short nail, namely a stationary fixing mode wherein the locking screw 5 is installed transversely close to the edge of the elongated locking screw hole 1-3 or installed obliquely from an outer upper part to an inner lower part, and a dynamic fixing mode wherein the locking screw 5 is installed transversely but not close to an upper edge.

In a further implementation, according to the differences of the femur bone conditions and fracture types among the patients, the threaded lag screws for the intramedullary nail can be used in combination as required as follows: a. for a stable fracture, the simplest combination including an intramedullary nail 1, a threaded lag screw 31, a locking screw 5, and a cap 6 may be selected, wherein the set screw 2 is in an unlocked state (i.e., it is elastically locked) and thus can only move rearwards, not forwards; b. if the restoration for a fracture is not good, compression is required at the first stage of the surgery, where a compression screw 4 is additionally provided within the overlapping hole 1-2 below the proximal end of the main nail to achieve a stable controllable compression operation during surgery; c. for a patient having a narrow femoral neck, a short screw in terms of length may be selected, such as a short compression screw 4 not passing through the femoral isthmus; and d. for a patient having a poor bone condition, a lag screw having bone-cement through-holes 36 at the head portion is selected, wherein bone cement is injected to achieve stabilization of the prosthesis and the patient's femur at the first stage.

According to the differences of the femur bone conditions and fracture types among the patients, the spiral-blade-type lag screws for the intramedullary nail can be used in combination as required as follows: a. for a stable fracture, the simplest combination including an intramedullary nail 1, a spiral-blade-type lag screw 31, a locking screw 5, and a cap 6 may be selected, wherein the set screw 2 is in an unlocked state (i.e., it is elastically locked) and thus can only move rearwards, not forwards; b. if the restoration for a fracture is not good, compression is required at the first stage of the surgery, wherein a compression screw 4 is additionally provided within the overlapping hole 1-2 below the proximal end of the main nail to achieve a stable controllable compression operation during surgery; c. for a patient having a narrow femoral neck, a short screw in terms of length may be selected, such as a short compression screw 4 not passing through the femoral isthmus; and d. for a patient having a poor bone condition, a spiral-blade-type lag screw having bone-cement through-holes 36 at the head portion is selected, wherein bone cement is injected to achieve stabilization of the prosthesis and the patient's femur at the first stage.

## Claims

1. An assembled intramedullary nail system, comprising: a main nail (1), a threaded lag screw, a spiral-blade-type lag screw, a long compression screw, a short compression screw, and at least one locking screw (5), wherein the main nail (1) comprises a center through-hole (1-1), an overlapping hole (1-2), and at least one locking screw hole (1-3) corresponding to the locking screw (5), wherein a side of the overlapping hole (1-2) adjacent to a proximal end is greater than the other side adjacent to a distal end, and wherein the threaded lag screw and the spiral-blade-type lag screw (3) can be interchangeably installed at one side of the overlapping hole (1-2) adjacent to the proximal end, the long compression screw and the short compression screw (4) can be interchangeably installed at one side of the overlapping hole (1-2) adjacent to the distal end, and the locking screw (5) can be disposed within the locking screw hole (1-3).

2. The assembled intramedullary nail system according to claim 1, wherein the main nail (1) is a long or short main nail.

3. The assembled intramedullary nail system according to claim 2, wherein three locking screws (5) are provided, and the main nail (1) comprises three locking screw holes (1-3).

4. The intramedullary nail system according to any of claims 1-3, wherein the threaded lag screw and the spiral-blade-type lag screw comprise bone-cement holes.

5. The assembled intramedullary nail system according to claim 4, further comprising a set screw (2) pre-installed within the center through-hole (1-1) of the main nail (1) and being adapted to cooperate with an engaging rail (34) of the lag screw (3).

6. The assembled intramedullary nail system according to claim 5, wherein, by driving the set screw (2), the threaded lag screw and the spiral-blade-type lag screw are allowed to be switched between a locked state and an unlocked state.

7. The assembled intramedullary nail system according to claim 6, wherein the threaded lag screw and the spiral-blade-type screw each comprises a lag thread (31) at a head portion and a compression rail (32) at a middle portion; wherein the long compression screw and the short compression screw each comprises a compression thread (41) at a head portion, and a tail portion of the long compression screw and the short compression screw being a cylinder (42); and wherein the compression threads (41) cooperate with the compression rail (32).

8. The assembled intramedullary nail system according to claim 7, wherein the threaded lag screw and the spiral-blade-type lag screw each comprises a driving slot (33) at the tail portion.

9. The assembled intramedullary nail system according to claim 8, wherein the compression rail (32) and the engaging rail (34) are respectively disposed at two sides of the threaded lag screw or the spiral-blade-type lag screw (3).

10. The assembled intramedullary nail system according to claim 9, further comprising a tail cap (6) which can be installed within the center through-hole (1-1) at the proximal end of the main nail (1).
